# EUROPEAN PATENT APPLICATION

(11) **EP 3 144 310 A1**
(43) Date of publication of application: **22.03.2017**
(21) Application number: 15792063.8
(22) Date of filing: 15.05.2015
(51) Int. Cl.: C07D 487/04, A61K 31/5377, A61K 31/454, A61K 31/4162, A61K 31/496, A61P 5/48, A61P 3/10

(54) **AMINOTETRAHYDROPYRAN DERIVATIVE USED AS DIPEPTIDYL PEPTIDASE-IV INHIBITOR**

(30) Priority: 15.05.2014 CN 201410205646
(71) Applicant: FUJIAN BORO MEDICAL CO., LTD., Fuzhou Fujian 350011 (CN)
(72) Inventor: LI, Dequn, Chengdu Sichuan 610213 (CN)
(74) Representative: Grünecker Patent- und Rechtsanwälte PartG mbB
(86) International application number: PCT/CN2015/079016
(87) International publication number: WO 2015/172732

(57) **Abstract**

Provided is an Aminotetrahydropyran derivative represented by general formula (I), a preparation method for the derivative, a pharmaceutical composition containing the derivative, and the use of the derivative to prepare a therapeutic agent, especially a dipeptidyl peptidase-IV inhibitor.

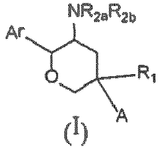

## Description

### TECHNICAL FIELD

The present invention relates to compounds for inhibiting dipeptidyl peptidase and the preparation method thereof, and the use of the compounds, alone or in combination with other medicament, in treating diseases related to the activity of dipeptidyl peptidase.

### TECHNICAL BACKGROUND

With the change of people's diet and life style, the number of diabetics is increasing. There are about 0.194 billion diabetics around the world, it is estimated that the number of diabetics will increase to 0.366 billion in the year 2030. For a long time, the treatment of type 2 diabetes mainly focus on small molecular oral medicines, sulfonylureas, Glinides, biguanides and thiazolidinediones are common medicines for treating type 2 diabetes. However, long term use of those medicines may induce adverse reactions such as hypoglycemia, body weight increase, damage of the function of β cells, therefore, the treatment with insulin or the combination with insulin is still used finally.

Glucagon-like peptide 1 (GLP-1) is an incretin, which accelerate the synthesis and secretion of insulin by stimulating and protecting islet β cells, and decreasing postprandial blood glucose. Dipeptidyl peptidase 4 (DPP-4) is a highly specific serine protease present in the form of a dimer, which can specifically recognize the alanine residue on the 2-position of the N terminal of GLP-1, and cut the dipeptide therefrom to deactivate GLP-1. The inhibition of DPP-4 can enhance the activity of GLP-1, and reduce the symptoms of high blood sugar of type 2 diabetics.

The development of DPP-4 inhibitors can be divided into 3 stages: the inhibitors developed in the first stage have strong inhibiting effect for DPP-4, but have poor selectivity to related enzymes such as DPP-7, DPP-8, DPP-9. Researches have shown that inhibition to DPP-7 will result in the death of T cells during dormancy; the inhibition to DPP-8 or DPP-9 of rats would result in bald, thrombocytopenia, reticulopenia, splenomegaly and pathological alteration of many organs and tissues etc., thus result in the increase in the mortality rate; the inhibition to the DPP-8 or DPP-9 of dogs would result in the toxicity of intestines and stomach; the inhibition to DPP-8 or DPP-9 of humans would decrease the activity of E cells, thus influence the immune function of the body. Therefore, the developments of new drugs require the compounds to have a high selectivity to DPP-4. The inhibitors developed in the second stage have a high inhibiting activity and a high selectivity to DPP-4, and generally have an IC50 to DPP-8 or DPP-9 above 1000 times of the IC50 to DPP-4. The inhibitors developed in the third stage have high activity and selectivity, and the acting time of the drug can last more than 24 hours.

In recent years, many DPP-4 inhibitors with new structures, strong effects and high selectivity have emerged, such as the sitagliptin phosphate developed by Merck Co., which appeared on the market in 2006 in the U.S.; vildagliptin developed by Novartis Co., which appeared on the marked in 2007 in Europe; saxagliptin developed by Bristol-Myers Squibb Co. and AstraZeneca Co., which appeared on the market in 2009 in the U.S; alogliptin benzoate of Takeda Pharmaceutical Co., which appeared on the market in 2010 in Japan; linagliptin of Boehringer-Ingelheim Pharmaceuticals, which appeared on the market in 2011 in the U.S.; teneligliptin developed by Mitsubishi Tanabe Pharmaceuticals. and Daiichi Sankyo Co., which appeared on the market in 2012 in Japan; gemigliptin of LG Life Sciences, which appeared on the market in 2012 in Korea; anagliptin of Sanwa Kagaku Kenkyushuo, which appeared on the market in 2012 in Japan. Besides, there are lots of drugs that are in the clinical trial stage.

Because DPP-4 inhibitors can inhibit the activity of DPP-4, it attracts extensive attention in the treatment of diabetes. Meanwhile, the development of DPP-4 inhibitors with high efficiency, low toxicity and high selectivity has become the study focus.

### SUMMARY OF THE INVENTION

The object of the present invention is to provide 3-aminotetrahydropyran compounds, the pharmaceutical acceptable salts, hydrates, solvates or stereisomers thereof as new DPP-4 inhibitors.

Another object of the present invention is to provide pharmaceutical compositions of the compounds of the present invention, the pharmaceutical acceptable salts, hydrates, solvates or stereisomers thereof.

Yet another object of the present invention is to provide the application of the compounds of the present invention in the preparation of the medicament for diseases related to DPP-4 enzyme activity.

One further object of the present invention is to provide a method for treating diseases related to DPP-4 enzyme activity with the compounds of the present invention, the pharmaceutical acceptable salts, hydrates, solvates, stereisomers thereof or compositions thereof.

In one aspect, the present invention provides a compound of formula (I), the pharmaceutical acceptable salts, hydrates, solvates or stereisomers thereof: 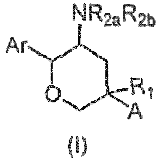
or the pharmaceutical acceptable salts, hydrates, solvates, stereisomers thereof; wherein Ar is a substituted or unsubstituted aryl, heteroaryl;
R₁ is hydrogen, a substituted or unsubstituted alkyl;
R₂ₐ and R_{2b} are each independently selected from hydrogen, or
R₂ₐ and R_{2b} are each independently selected from substituted or unsubstituted alkyl, alkoxy, cycloalkyl, heterocyclylalkyl; or
R₂ₐ and R_{2b} form heterocyclylalkyl with the nitrogen atom attached to them, and the heterocyclylalkyl can be optionally substituted;
A is selected from: 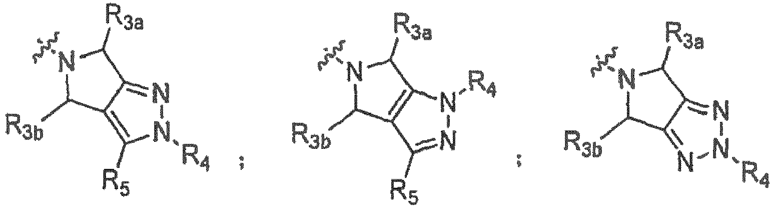 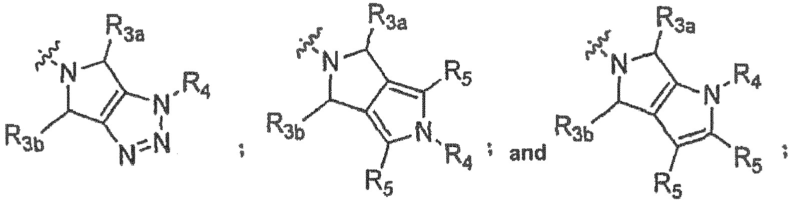
R₃ₐ and R_{3b} are each independently selected from hydrogen, substituted or unsubstituted C₁₋₁₀ alkyl;
R₄ is: 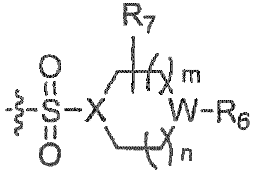
X is CR₈ or N;
wherein 0-3 R₇ may be present, and each R₇ is independently oxo, halogen, substituted
or unsubstituted alkyl, substituted or unsubstituted alkoxy, or two or more R₇ form brideged alkyls;
W is CR₈, O, or N;
R₆ is selected from H, hydroxyl, or
R₆ is selected from substituted or unsubstituted C₁₋₈ alkyl, C₁₋₈ alkyl-R₉, C₃₋₁₄ cycloalkyl, 3-14 membered heterocyclylalkyl, C(O)-C₁₋₈ alkyl, C₁₋₈ halogen substituted alkyl, C₁₋₈ alkyl hydroxyl, C(O)NR₉R₁₀, C₁₋₈ cyano alkyl, C(O)R₉, C₀₋₈ alkyl-C(O)-C₀₋₈ alkyl-NR₉R₁₀, C₀₋₈ alkyl-C(O)OR₉, NR₉R₁₀, SO₂-C₁₋₈ alkyl, C₁₋₈ alkyl-C₃₋₁₄ cycloalkyl, C(O)-C₁₋₈ alkyl-C₃₋₁₄ cycloalkyl, C₁₋₈ alkoxy;
R₈ is a hydrogen or halogen;
R₉ and R₁₀ are each independently selected from H, C₁₋₈ alkyl, C₃₋₁₄ cycloalkyl, 3-14 membered heterocyclylalkyl, C₆₋₁₄ aryl, 5-14 membered heteroaryl, alkoxy, C(O)C₁₋₄alkyl, C₁₋₈ alkylamino, C₁₋₆ alkyls hydroxyl;
m is 0, 1 or 2;
n is 0, 1 or 2;
R₅ is selected from H, halogen, cyano, amino, nitro, or
R₅ is selected from substituted or unsubstituted C₁₋₁₀ alkyl, C₁₋₁₀ alkoxy, C₂₋₁₀ alkenyl, (CH₂)ₚ-aryl, (CH₂)ₚ-heteroaryl, (CH₂)ₚ-cycloalkyl, (CH₂)ₚ-heterocyclylalkyl, (CH₂)ₚ-NR₁₁R₁₂, (CH₂)ₚ-SO₂NR₁₁R₁₂, (CH₂)ₚ-SO₂R₁₃, (CH₂)ₚ-NR₁₁SO₂R₁₃, (CH₂)ₚ-OR₁₃, (CH₂)ₚ-OCOR₁₃, (CH₂)ₚ-OCONR₁₁R₁₂, (CH₂)ₚ-CONR₁₁R₁₂, (CH₂)ₚ-NR₁₃CONR₁₁R₁₂, (CH₂)ₚ-COOH, (CH₂)ₚ-COR₁₃, (CH₂)ₚ-CO₂C₁₋₆alkyl, (CH₂)ₚ-NR₁₁COOR₁₃;
wherein, R₁₁ and R₁₂ are independently selected from hydrogen, (CH₂)_{q}-phenyl, (CH₂)_{q}-C₃₋₈ cycloalkyl, C₁₋₆ alkyl, wherein alkyl is optionally substituted by 1-5 substituents selected from fluorine or hydroxyl, wherein phenyl and cycloalkyl are optionally substituted by 1-5 substituents independently selected from halogen, hydroxyl, trifluoromethyl, C₁₋₆ alkyl or C₁₋₆ alkoxy;
or R₁₁ and R₁₂, together with the nitrogen atom they are attached to, form a heterocyclic ring selected from piperidine, piperazine, morpholine, pyrrole or azetidine, wherein the heterocyclic ring is optionally substituted by 1-3 substituents independently selected from halogen, hydroxyl, C₁₋₆ alkyl or C₁₋₆ alkoxy;
each R₁₃ is independently C1-6 alkyl, wherein the alkyl is optionally substituted by 1-5 substituents selected from fluorine or hydroxyl;
p is 0, 1, 2, 3, 4, 5 or 6;
q is 0, 1 or 2.

In one example of the compounds of the present invention, Ar is a phenyl that is optionally substituted by 1-5 R₁₄;
each R₁₄ is independently selected from halogen, hydroxyl, cyano, nitro, nitroso, amino, imino, carboxyl, sulfydryl or
each R₁₄ is independently selected from substituted or unsubstituted alkyl, acyl, acylamino, ester group, acylester group, phenoxy, benzyl, benzyloxy, sulfonyl, sulfinyl, cycloalkyl, heterocyclylalkyl, alkenyl, alkynyl, alkoxy, allyloxy, alkylamino, aryl, heteroaryl.

In one such example, Ar is a phenyl that is optionally substituted by 1-3 substituents independently selected from F, Cl, Br, I, -CH₃, -CF₃ and -OCF₃.

In another such example, Ar is 2,5-difluorophenyl or 2,4,5-trifluorophenyl.

In a second example of the compounds of the present invention, R₂ₐ and R_{2b} are each independently selected form hydrogen, C₁₋₁₀ alkyl, alkoxy, C₃₋₁₄ cycloalkyl, 3-14 membered heterocyclylalkyl;
wherein, alkyl is optionally substituted by 1-6 substituents that are independently selected from halogen, hydroxyl, trifluoromethyl;
alkoxyl is optionally substituted by 1-6 substituents that are independently selected from halogen or hydroxyl;
cycloalkyl is optionally substituted by 1-3 substituents that are independently selected from halogen, hydroxyl, cyano, nitro, carboxyl, C₁₋₆ alkyl, C₁₋₆ alkyloxycarbonyl or C₁₋₆ alkoxyl, wherein alkyl and alkoxyl can be substituted by 1-5 fluorine;
heterocyclylalkyl is optionally substituted by 1-3 substituents that are independently selected from oxo, halogen, hydroxyl, cyano, nitro, carboxyl, C₁₋₆ alkyl, C₁₋₆ alkyloxycarbonyl or C₁₋₆ alkoxyl;
or R₂ₐ and R_{2b}, together with the nitrogen atom they are attached to, form a heterocyclic ring that is selected from piperidine, piperazine, morpholine, pyrrole or azetidine, wherein the heterocyclic ring is optionally substituted by 1-3 substituents that are independently selected from halogen, hydroxyl, C₁₋₆ alkyl or C₁₋₆ alkoxyl, wherein each alkyl and alkoxyl are optionally substituted by 1-5 fluorine.

In one such example, R₂ₐ and R_{2b} are each independently selected from hydrogen, C₁₋₆ alkyl that is optionally substituted by 1-3 fluorine or hydroxyl; or R₂ₐ and R_{2b}, together with the nitrogen atom they are attached to, form a heterocyclic ring that is selected from piperidine, piperazine, morpholine, pyrrole or azetidine.

In another such example, R₂ₐ and R_{2b} are hydrogens.

In a third example of the compounds of the present invention, R₃ₐ and R_{3b} are independently selected from hydrogen, C₁₋₆ alkyl that is optionally substituted by 1-6 fluorine.

In one such example, R₃ₐ and R_{3b} are hydrogens.

In a fourth example of the compounds of the present invention, A is:

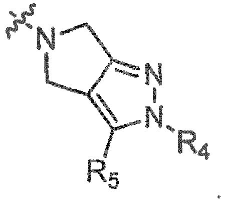

In the subclass of the fourth example, R₅ is hydrogen.

In a fifth example of the compounds of the present invention, R₄ is: 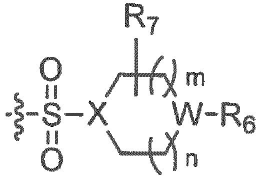
X is CR₈ or N;
wherein 0-2 R₇ may be present, and R₇ is oxo, or two R₇ form brideged alkyl;
W is CR₈, O, or N;
R₆ is selected from H, hydroxyl, or
R₆ is selected from substituted or unsubstituted C₁₋₈ alkyl, C₃₋₈ cycloalkyl, 3-8 membered heterocyclylalkyl, C(O)-C₁₋₆ alkyl, C(O)NR₉R₁₀, C(O)R₉, NR₉R₁₀, SO₂-C₁₋₈alkyl;
R₈ is a hydrogen or halogen;
R₉ and R₁₀ are each independently selected from H, C₁₋₆ alkyl, C₃₋₈ cycloalkyl, 3-8 membered heterocyclylalkyl;
m is 0, 1, or 2;
n is 0, 1 or 2.

In a sixth example of the compounds of the present invention, the compounds of formula Ia and Ib with the shown stereochemical configuration are provided, which have trans-form Ar and NR₂ₐR_{2b} substituents on the two * marked tetrahydropyran carbon atoms that are formed stereoscopically: 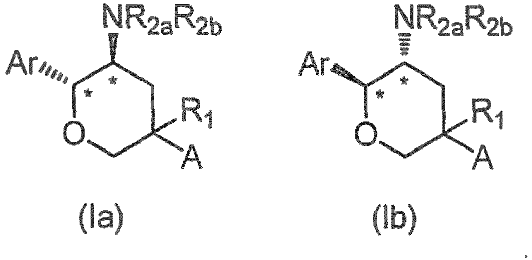 Wherein Ar, NR₂ₐR_{2b} and A are defined as above.

In one such example, compounds of formula la with the shown stereochemical configuration are provided, which have trans-form Ar and NR₂ₐR_{2b} substituents on the two * marked tetrahydropyran carbon atoms that are formed stereoscopically:

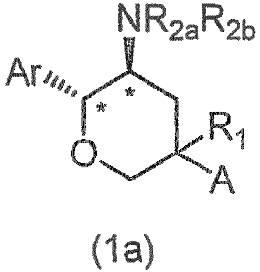

In another such example, compounds of formula Ic and Id with the shown absolute stereochemical configuration are provided, which have trans-from Ar and NR₂ₐR_{2b} substituents, trans-form Ar and A substituents and cis-form NR₂ₐR_{2b} and A substituents on the three * marked tetrahydropyran carbon atoms that are formed stereoscopically:

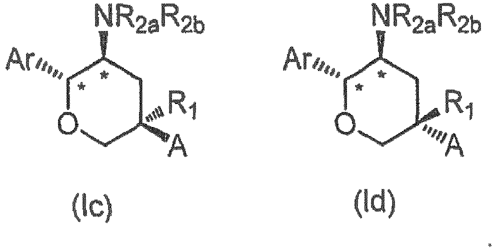

In the subclass of the sixth example, compounds of formula Ic with the shown absolute stereochemical configuration are provided, which have trans-from Ar and NR₂ₐR_{2b} substituents, trans-form Ar and A substituents and cis-form NR₂ₐR_{2b} and A substituents on the three * marked tetrahydropyran carbon atoms that are formed stereoscopically:

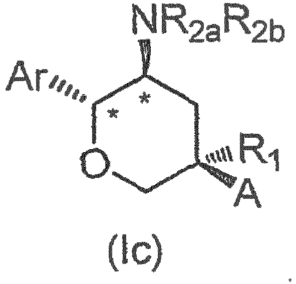

In the subclass of such examples, A is selected from: 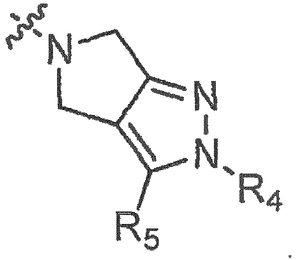 wherein R₄ and R₅ are defined as above.

In the subclass of such examples, R₅ is H.

Typical compounds of the present invention comprise, but are not limited to:

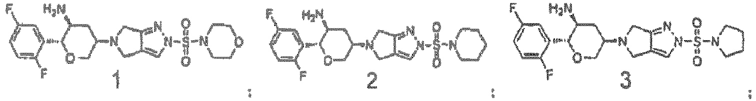

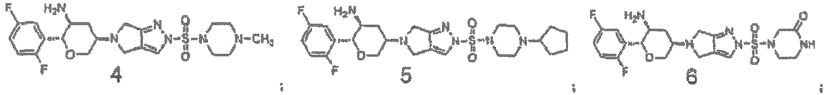

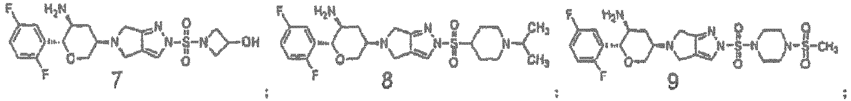

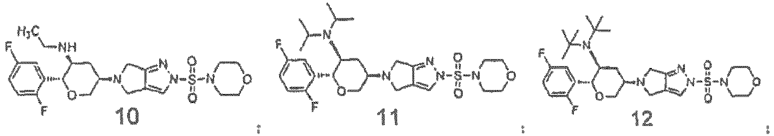

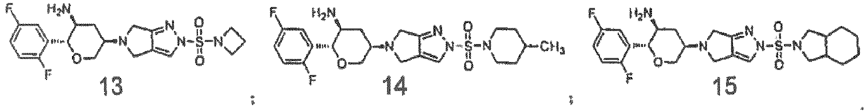

The compounds of formula (I) have chiral centers, therefore, there might be different enantiomers and diastereomers. Specifically, in the form Ia, Ib, Ic and Id, the compounds of the present invention have a * marked carbon atom asymmetric center, each of the asymmetric center will independently construct two optical isomers. All possible mixtures of optical isomers and diastereoisomers and pure or partially purified compounds are included in the extent of the present invention.

The present invention comprise compounds that are marked with isotopes, they are equal to those described by formula (I), but one or more atoms are replaced by atoms with atom mass or mass number that are different from that of atoms that are common in natural. Examples of isotopes that can be introduced into the compounds of the present invention comprise isotopes of hydrogen, carbon, nitrogen, oxygen, phosphorus, sulfur, fluorine and chlorine, respectively, for example, ²H, ³H, ¹³C, ¹¹C, ¹⁴C, ¹⁵N, ¹⁸O, ¹⁷O, ³¹P, ³²P, ³⁵S, ¹⁸F and ³⁶Cl. The compounds of the present invention that comprise the above isotopes and/or other isotopes of other atoms, the pro-drugs thereof and the pharmaceutical acceptable salts of the compounds and the pro-drugs all belong to the extent of the present invention. Some of the isotope-marked compounds of the present invention, such as those that introduce radioactive isotopes (such as ³H and ¹⁴C), can be used for the measurement of the distribution of drug and/or substrate tissue. Tritium, i.e. ³H and Carbon-14, i.e. ¹⁴C isotope are especially preferred, as they can be easily prepared and tested, and further, can be replaced by heavier isotopes, such as deuterium, i.e. ²H, and can bring benefits in treatment because of their higher metabolic stability, such as prolonging the half-life in vivo or decreasing the requirement for the dosage, therefore, they are preferred in some circumstance. The isotope marked-compound of formula (I) of the present invention and the pro-drug thereof generally can be prepared as follows: when carrying out the following process and/or the process disclosed in the examples and preparation examples, using the isotope marked-reagents that are easy to obtain to replace the reagents that are marked by elements that are not isotopes.

The compounds of formula (I) can further be formed formulations that are pharmaceutically acceptable, comprising the salts, solvates of the compounds of formula (I), the salts comprise but are not limited to acid addition salts and/or alkali salt.

The present invention also provides pharmaceutical reagents, comprising effective amount of compounds of formula (I) or the pharmaceutical salts thereof and the pharmaceutical acceptable carriers, diluents or excipients thereof. All of these forms belong to the present invention.

The following definitions used herein are suitable.

"alkyl" represents one group or other groups in the present invention, such as structure unit that is halogen substituted alkyl and alkoxyl, and can be linear or branched, comprising linear alkyl such as methyl, ethyl, propyl, butyl, pentyl, hexyl, heptyl, octyl, nonyl, decyl, undecyl, dodecyl and so on. The term also comprises branched isomers of linear alkyls, comprising but are not limited to the following groups: -CH(CH₃)₂, -CH(CH₃)(CH₂CH₃), -CH(CH₂CH₃)₂, -C(CH₃)₃, -CH₂CH(CH₃)₂, -CH₂CH(CH₃)(CH₂CH₃), -CH₂CH(CH₂CH₃)₂, -CH₂C(CH₃)₃, -CH₂C(CH₂CH₃)₃, -CH(CH₃)-CH(CH₃)(CH₂CH₃), -CH₂CH₂CH(CH₃)₂, -CH₂CH₂CH(CH₃)(CH₂CH₃), -CH₂CH₂CH(CH₂CH₃)₂, -CH₂CH₂C(CH₃)₃, -CH₂CH₂C(CH₂CH₃)₃, -CH(CH₃)CH₂CH(CH₃)₂, -CH(CH₃)CH(CH₃)CH(CH₃)₂, -CH(CH₂CH₃)CH(CH₃)CH(CH₃)(CH₂CH₃) and so on.
Therefore, the term alkyl comprises primary alkyls, secondary alkyls and tertiary alkyls.
Preferred alkyls comprise C₁₋₁₂ linear alkyl and C₃₋₁₀ branched alkyl.

The term "alkenyl" represents alkyls that are defined above with two adjacent carbon atoms that are connected with a double bond.

The term "alkynyl" represents alkyls that are defined above with the two adjacent carbon atoms that are connected with a triple bond.

The term "halogen" represents fluorine, chlorine, bromine, iodine.

The term "alkoxy" represents (alkyl)O-groups, wherein alkyl is defined herein.

The term "hydroxyl" represents -OH.

The term "amino" represents -NH₂. The term "imino" represents -NH-.

The term "nitro" represents -NO₂. The term "nitroso" represents -NO.

The term "trifluoromethyl" represents -CF₃.

The term "carboxyl" represents -COOH.

The term "sulfydryl" represents -SH.

The term "aryl" represents aromatic ring with each atom that forms the ring is a carbon atom. Aromatic ring can be formed by 5, 6, 7, 8, 9, or more than 9 carbon atoms. Aryl can be optionally substituted. Examples of aryl comprise but are not limited to phenyl, naphthyl, phenanthryl, anthryl, fluorenyl and indenyl.

The term "heteroaryl" or optionally "heteroaromatic group" represents aromatic group comprising one or more heterocyclic atoms selected from nitrogen, oxygen and sulphur.
The "heteroaromatic group" or the "heteroaryl" that comprises N atom represents an aromatic group, wherein one of the atoms in the backbone of the ring is nitrogen. The illustrative examples of heteroaryl groups comprising the following parts: 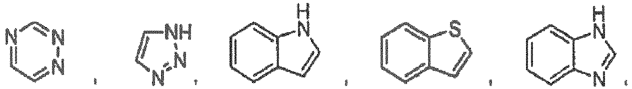 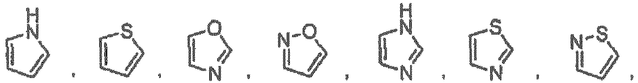 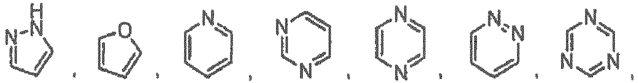 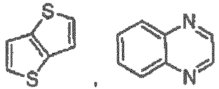 and so on. According to the structures, heteroaryl can be monoradical or biradical (i.e., heteroarylene group).

The term "cycloalkyl" represents monocyclic or a polycyclic radical that only comprises carbon and hydrogen, and optionally being saturated, partially unsaturated, or completely unsaturated. Cycloalkyl group comprises a group having 3 to 10 ring atoms. The illustrative examples of cycloalkyl groups comprising the following parts: 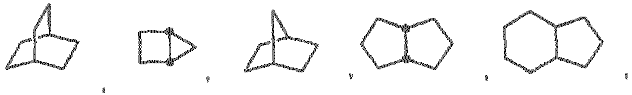 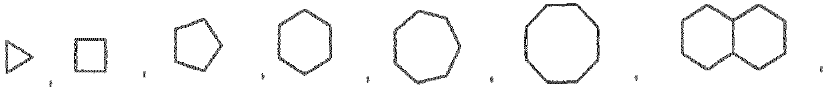 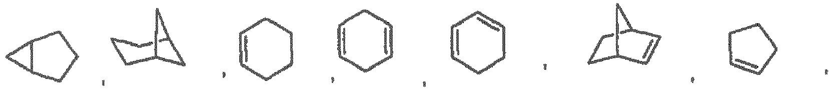 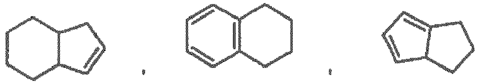 and so on. According to the structures, the cycloalkyl groups can be monoradical or biradical (e.g., cycloalkylene group), if it is a "lower cycloalkyl", it has 3-8 carbon atoms.

The term "heterocyclylalkyl" represents cycloalkyls that do not belong to aromatic cycloalkyls but comprise at least one heteroatom selected from O, N and S and optionally comprise one or more double bonds or triple bonds. Heterocyclylalkyl as a whole can has 3-14 ring atoms and 1-5 heteratoms (for example, monocyclic heterocyclylalkyl has 3-6 ring atoms, polycyclic heterocyclylalkyl has 7-14 ring atoms). On the heteroatoms or carbon atoms that form stable structures, heterocyclylalkyl can be covalently attached to the defined chemical structure. The N or S atoms on heterocyclylalkyl can be oxidized (such as morpholine N-oxide, thiomorpholine S-oxide, thiomorpholine S, S-dioxide). Heterocyclylalkyl can comprise one or more oxo groups, such as phthalimido, piperidinonyl, oxazolidinonyl, 2,4(1H, 3H)-dioxo-pyrimidyl, pyridine-2(1H)- ketone group and so on. The examples of heterocyclylalkyl also comprise morpholinyl, thiomorpholinyl, pyranyl, imidazolidinyl, imidazolinyl, oxazolidinyl, isoxazolidinyl, pyrazolidinyl, pyrazolinyl, pyrrolidyl, pyrrolinyl, tetrahydrofuranyl, tetrahydrothienyl, thiazolidinyl, octahydroisoindolyl, piperidyl, azetidine, N-methylazetidinyl, piperazinyl, N-methyl piperazinyl, diazepinyl and so on.

The term "oxo" represents an oxygen with a double bond (i.e., =O).

The term "acyl" represents -CO- group, illustrative acyl comprises acetyl, trifluoroacetyl, benzoyl and so on.

The term "acylamino" represents a chemical part with the formula -C(O)NHR or -NHC(O)R, wherein R is selected from alkyl, cycloalkyl, heterocyclylalkyl (bonded through a ring carbon atom), aryl, heteroaryl (bonded through a ring carbon atom).

The term "ester group" represents a chemical part with the formula -COOR, wherein R is selected from alkyl, cycloalkyl, heterocyclylalkyl (bonded through a ring carbon atom), aryl, heteroaryl (bonded through a ring carbon atom).

The term "acyl ester group" represents a chemical part with the formula -CO-COOR, wherein R is selected from alkyl, cycloalkyl, heterocyclylalkyl (bonded through a ring carbon atom), aryl, heteroaryl (bonded through a ring carbon atom).

The compounds of the present invention can also be administered in the form of a pharmaceutically acceptable salt. The term "pharmaceutically acceptable salt" refers to salts prepared from pharmaceutically acceptable non-toxic bases or acids including inorganic or organic bases and inorganic or organic acids. Basic compounds encompassed within the term "pharmaceutically acceptable salt" refer to non-toxic salts of the compounds of this invention which are generally prepared by reacting the free base with a suitable organic or inorganic acid.

### EMBODIMENTS

The method for preparing the compounds of the present invention:
The compounds of formula (I) can be prepared according to process 1, wherein every substituent is defined as in SUMMARY OF THE INVENTION:

### Process 1

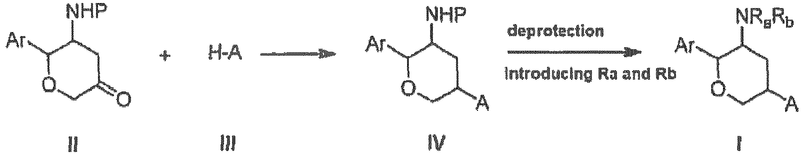

In the presence of intermediate III, reagents such as sodium borohydride, decaborane or sodium triacetoxyborohydride are used in suitable solvents such as tetrahydrofuran, methanol, methylbenzene, pyridine, DMF or DMSO to provide intermediate IV through the reductive amination of the intermediate II. The reaction is carried out under the presence of Lewis acids such as titanium tetrachloride or titanium tetraisopropylate. The reaction can also be accelerated by adding acid such as acetic acid. In certain circumstance, the intermediate III can be a salt, such as trifluoroacetate or hydrochloride, base such as N,N- diisopropylethylamine or triethylamine can be conveniently added to the reaction mixture. Then deprotection is carried out, such as for Boc, trifluoroacetic acid or hydrochloric acid-methanol solution is used, or for Fmoc, triethylamine, diethylamine, piperidine or morpholine is used for a reaction in DMF, NMP or acetonitrile, then Rₐ and R_{b} groups (when Rₐ and R_{b} are both not hydrogen) are introduced via a suitable method (such as under basic condition, the intermediate IV is reacted with RₐCl and/or R_{b}Cl) to obtain the compound of formula (I).

### Process 2

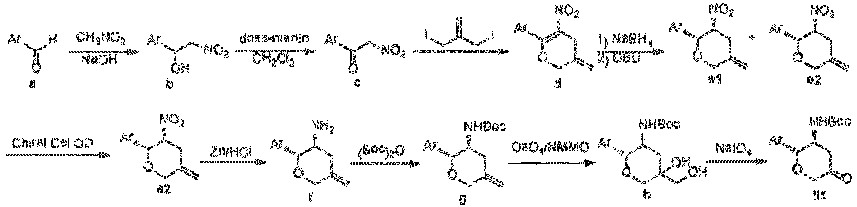

The intermediate II can be obtained by methods known by those skilled in the art. Nitroalcohol b is obtained via aldehyde a and nitromethane under the presence of a base, then the obtained nitroalcohol b is treated with a oxidizing agent such as Dess-martin, to obtain nitroketone c. d is prepared through the reaction of nitroketone c and 3-iodo-2-(iodomethyl) prop-1-ene under heating. The obtained d is reduced by a reducing agent such as sodium borohydride to obtain two products: cis-product and trans-product, wherein the cis-product ban be easily transformed into the trans-product by isomerization of a base such as DBU. The obtained trans products e1 and e2 are separated through HPLC via a chiral cel to obtain e2. Then, e2 is reduced by zinc and acid such as hydrochloric acid to obtain aminopyran f, g is produced by the protection of amino by a protecting group such as Boc. Dihydroxyl compound h is obtained by the treatment of oxidizing agents such as osmium tetroxide and N-methylmorpholine N-oxide, then the intermediate IIa is obtained by the oxidizing of sodium periodate.

### Process 3

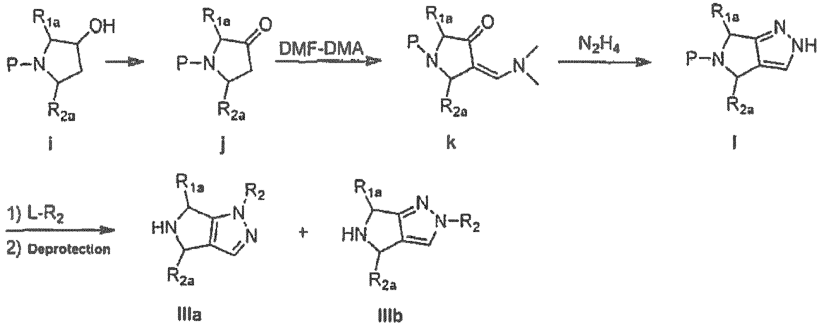

The intermediate III can be prepared by a general process 3. The pyrrolidinol i protected by Boc or other protecting agent can be transformed into pyrrolidone j through oxidization such as Swern oxidization. Under the presence of DMF-DMA, k is provided by j under heating, then k is used to obtain intermediate I in a suitable solvent such as ethanol with the presence of hydrazine under heating. I is reacted with L-R₂, after L leaves, the intermediates IIIa and IIIb are provided via deprotection under a suitable condition, and IIIa and IIIb ban be separated by methods known by those skilled in the art such as column chromatography.

The purposes of the following examples are to describe specific embodiments of the invention, but not limit the extent of the description and claims. Those skilled in the art can understand that the raw materials can be different, and the compounds emcompassed by the present invention can be produced via additional steps as shown in the following examples. The following examples are only provided for illustrating purpose, they are not intended to or should be understood in any ways to limit the present invention. Those skilled in the art should realize that changes and modifications without violating the spirit or extent of the present invention can be accepted.

### Abbreviation List

- DMF:: N,N-dimethylformamide
- DMSO:: dimethyl sulfoxide
- Boc:: t-butyloxycarbonyl
- Fmoc:: fluorene methoxyl carbonyl acyl
- NMP:: N- methyl pyrrolidone
- DBU:: 1,8-diazacyclo[5,4,0] hendecene-7
- HPLC:: High Performance Liquid Chromatography
- DMF-DMA:: N,N- dimethylformide dimethyl acetal
- DIPEA:: N,N-diisopropylethylamine
- NMO:: N-methyl-N-morpholine oxide

### Example 1:

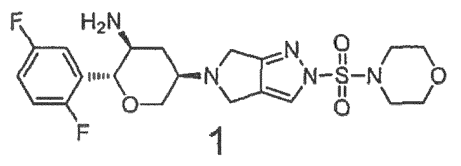

### Preparation of compound 1: (2R, 3S, 5R)-2-(2,5-difluorophenyl)-5-[2-(morpholine sulfonyl)-2,6-dihydropyrrolo[3,4-c] pyrazol-5(4H)-yl]tetrahydro-2H-pyran-3-amine

### A. Preparation of intermediate

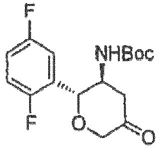

### Step 1. Preparation of 1-(2,5-difluorophenyl)-2-nitroethanol

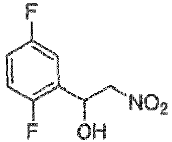

At 5°C, 50mL methanol solution of 2,5- difluorobenzaldehyde (50g, 0.35mol) and nitromethane (22.4mL, 0.414mol) were added to the methanol (214mL) solution of sodium hydroxide (1 N, 428mL) (more than 1h), after the addition, glacial acetic acid (24mL) was added to neutralize the reaction solution, then diethyl ether was added, the organic layer was washed with saturated sodium carbonate (30mL) and saturated salt water (30mL), the organic layer was dried over anhydrous magnesium sulfate and was concentrated to obtain 1-(2,5-difluorophenyl)-2-nitroethanol, and the product can be used in step 2 without further purification.

### Step 2. Preparation of 2-nitro-1-(2,5-difluorophenyl) ethanone

At 10°C, within 30 minutes, the dess-martin periodinane (184g) was added into the dichloromethane solution of nitroethanol (68.1g) prepared in step 1 in three batches. Continuously stirred for 24h, then the reaction mixture was poured to the mixing solution of sodium bicarbonate (441g) and sodium thiosulfate (489g) in water (4.2L). HCl (6N, 1.5L) was used to neutralize the aqueous layer until no more bubble was generated, extracted with dichloromethane, washed one time with sodium bicarbonate solution. The organic layer was dried over anhydrous magnesium sulfate then combined, filtered and evaporated to dryness, purified through chromatography (silica gel, petroleum ether : ethyl acetate 10:1-6:1 gradient elute) to obtain the desired nitroketone 54g. ¹H NMR (400MHz, CDCl₃) δ 7.74-7.70(m, 1 H), 7.43-7.37(m, 1 H), 7.28-7.22(m, 1 H), 5.82(d, J= 3.6Hz, 2H).

### Step 3. Preparation of 3-iodo-2-(iodomethyl)-isopropyl-1-ene

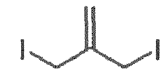

The reaction solution of 3-chloro-2-(chloromethyl)prop-1-ene (5.75mL, 54.7mmol) and sodium iodide (45g, 301mmol) in acetone (400mL) was stirred at room temperature for 20h, and concentrated to dry under vacuum, then dispersed in dichloromethane and water. The organic layer was dried over anhydrous sodium sulfate, filtered, evaporated to dryness to obtain 3-iodo-2-(iodomethyl)prop-1ene, which is a light red oily compound.

### Step 4. Preparation of 6-(2,5-difluorophenyl)-3-methylene-5-nitro-3,4-dihydro-2H-pyran

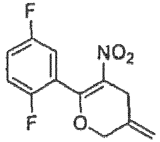

3-iodo-2-(iodomethyl)-isoprop-1-ene (16.8g, 54.7mmol) was dissolved in 50mL DMF, 20mL DIPEA was added, then 2-nitro-1-(2,5-difluorophenyl) ethanone (10g, 49.7mmol) was added slowly drop by drop, then was stirred for 2h at room temperature after the addition, 400mL water was added, extracted with ethyl acetate for three times, the organic layers were combined and dried over anhydrous sodium sulfate, 3g product was obtained by column chromatography (petroleum ether : dichloromethane 20%-30% gradient elute). ¹H NMR(400MHz, DMSO-d₆)δ7.50-7.29(m, 3H), 5.41(brs, 1H), 5.34(brs, 1 H), 4.74(s, 2H), 3.58(brs, 2H)

### Step 5. Preparation of (2R, 3S)-2-(2,5-difluorophenyl)-5-methylene-3-nitrotetrahydro-2H-pyran

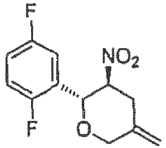

Silica gel (100-200 mesh) 58g and sodium borohydride (5.2g, 136.6mmol) were added to the solution of 6-(2,5-difluorophenyl)-3-methylene-5-nitro-3,4-dihydro-2H-pyran (9.1g, 35.96mmol) in chloroform (480ml) and isopropanol (90ml), stirred at room temperature overnight. Then diluted hydrochloric acid (69mL, 2N) was added slowly to quench the reaction, filtered. The silica gel was washed with ethyl acetate. The organic layer was washed with sodium bicarbonate, dried over anhydrous sodium sulfate, and concentrated to dry under vacuum, then subjected to column chromatography (petroleum ether:diethyl ether=16:1 to 15:1 gradient elute) to obtain trans-2-(2,5-difluorophenyl)5-methylene-3-nitrotetrahydrofuran-2H-pyran 5.5g and cis-2-(2,5-difluorophenyl)-5-methylene-3-nitrotetrahydrofuran-2H-pyran 2.4g. The product with the cis configuration was dissolved in 50mL tetrahydrofuran, 120 microlitre DBU was added. Stirred for 0.5h under room temperature then subjected to column chromatography (petroleum ether:diethyl ether=16:1 to 15:1 elute) to obtain 1.6g trans product. A part of trans product (1g) was separated by HPLC (ChiralCel OD) to obtain enantiomer (2R, 3S)-5-methylene-3-nitro-2-(2,4,5-trifluorophenyl)tetrahydro-2H-pyran 440mg. ¹H NMR(400MHz, CDCl₃)δ7.17-7.13(m, 1H), 7.06-7.03(m, 2H), 5.14-5.11(m, 2H), 5.08(d, *J*=9.6Hz, 1 H), 4.82-4.73(m, 1 H), 4.39(d, *J*=12.7Hz, 1 H), 4.24(d, *J*=12.8Hz, 1 H), 3.13(d, *J*=7.9Hz, 2H).

### Step 6. Preparation of (2R, 3S)-2-(2,5-difluorophenyl)-5-methylenetetrahydro-2H-pyran-3-amino

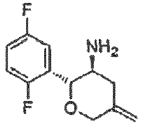

The product (440mg, 2.2mmol) obtained from the last step was dissolved in ethanol, zinc powder (1.2g, 18.9mmol) was added, 6N hydrochloric acid (6mL, 36mmol) was added under vigorous stirring. After 3h, the pH was adjusted to 10 with 2N sodium hydroxide, extracted with diethyl ether for three times, dried over anhydrous sodium sulfate, evaporating diethyl ether under vacuum to obtain colorless and clear oily liquid 376mg, then the next reaction was carried out directly.

### Step 7. Preparation of [(2R, 3S)-5-methylene-2-(2,5-difluorophenyl)tetrahydro-2H-pyran-3-yl]tert-butyl carbamate

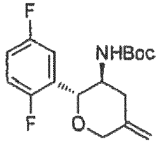

The colorless and clear oily liquid obtained from the last step was dissolved in dichloromethane, di-tert-butyl dicarbonate ester (507mg, 2.34mmol) was added, stirred at room temperature overnight, evaporating dichlormethane under vacuum to obtain 464mg product, then the reaction of next step was carried out directly.

### Step 8. Preparation of [(2R, 3S)-5-hydroxy-5-(hydroxy methyl)-2-(2,5-difluorophenyl)tetrahydro-2H-pyran-3-yl] tert-butyl carbamate

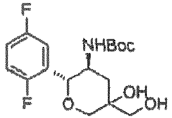

The product obtained from step 7 was dissolved in 7mL acetone, 3.5mL water and 14mL tertiary butanol were added, then 0.258mL 2.5% tertiary butanol solution of osmium tetroxide was added. Stirred at room temperature for 10 minutes, then 0.184mL NMO was added, reacted at room temperature for 48h, saturated sodium thiosulfate solution was added, stirred for half an hour, then extracted with ethyl acetate, dried over anhydrous sodium sulfate, concentrated to dry under vacuum to obtain 510mg product, then the reaction of the next step was carried out directly.

### Step 9. Preparation of [(2R, 3S)-5-oxo-2-(2,5-difluorophenyl)tetrahydro-2H-pyran-3-yl] tert-butyl carbamate

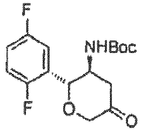

The product of the last step was dissolved in 10mL tetrahydrofuran, 4mL water solution of sodium periodate (327mg, 1.53mmol) was added, the mixture was stirred for 4h and was subjected to column chromatography (10% to 20% ethyl acetate in chloroform) after concentration to obtain 410mg white solid. ¹H NMR(400MHz, CDCl₃) δ 7.23(d, *J*=3.3Hz, 1H), 7.05-7.00(m, 2H), 4.83(d, *J*=8.0Hz, 1H), 4.71(d, *J*=9.0Hz, 1H), 4.31(dd, *J*=16.3, 1.5Hz, 1H), 4.18-3.99(m, 2H), 3.06(dd, *J*=16.6, 5.1Hz, 1H), 2.75-2.68(m, 1H), 1.31(s, 9H); ESI-MS:272.1(M+1-56).

### B Preparation of intermediate

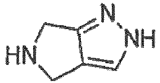

### Step 1. Preparation of

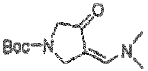

3-oxopyrrolidine-1-tert-butyl carboxylate (40g, 216mmol) was dissolved in 300mL DMF-DMA, heated for 40 minutes at 105°C. Cooled and concentrated to dry under vacuum, treated with hexane, filtered to obtain orange solid 41g, dried, the product can be used in the reaction of the next step without purification.

### Step 2. Preparation of

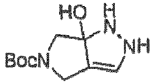

41 g orange solid obtained from the last step was dissolved in 200mL methylbenzene, 15mL hydrazine hydrate (80% concentration) was added slowly drop by drop at 40°C, reacted for 2h at room temperature after addition, the reaction solution was cooled with white solid separated out, the solid obtained via filtration was washed with n-heptane to obtain 37.2g product.

### Step 3. Preparation of

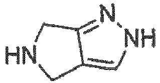

37.2g white solid obtained from the last step was dissolved in methanol, 5N hydrochloric acid was added, stirred at room temperature for 6h, ammonia methanol solution (2N) was added after concentration, the pH was adjusted to a slightly alkaline state, 200mL dichloromethane was added, 40mL triethylamine was added, dichloromethane solution (40mL) of 64g di-tert-butyl dicarbonate was added drop by drop at room temperature, after the reaction was carried out for half an hour, the solution was concentrated to dry under vacuum, then subjected to column chromatography (methanol: aqueous ammonia: ethyl acetate=135:15:350) to obtain 20g white solid. ¹H NMR(400MHz, CDCl₃) δ 8.03(brs, 1H), 7.34(d, *J*=12.8Hz, 1H), 4.50(t, *J*=16.6Hz. 4H), 1.53(s, 9H).

### C Preparation of intermediate

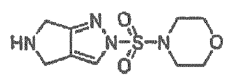

### Step 1. Preparation of

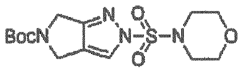

N-Boc pyrazolopyrolidine (100mg, 0.478mmol) was dissolved in 3mL anhydrous tetrahydrofuran, sodium hydride (30mg, 0.718mmol, 60% dispersion in oil) was added and stirred at room temperature for 1 h, then morpholinesulfonyl chloride (133.23mg, 0.718mmol) was added, reacted overnight, the solution was concentrated to dry under vacuum, water and ethyl acetate was added, after the organic layer was dried then was subjected to column chromatograph (petroleum ether: ethyl acetate=1:1) to obtain 147mg oily liquid. ¹H NMR(400MHz, CDCl₃) δ 7.67(d, *J*=15.4Hz. 1H), 4.49-4.39(m, 4H), 3.73-3.66(m, 4H), 3.34-3.21 (m, 4H), 1.47(s, 9H).

### Step 2. Preparation of

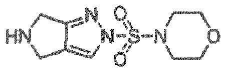

4mL dichloromethane and 1 mL trifluoroacetic acid were added to the product of the last step, after 2h of reaction at room temperature, concentrated to dry under vacuum to obtain 80mg product, the reaction of the next step can be carried out directly without purification.

### D. Preparation of compound 1: (2R, 3S, 5R)-2-(2,5-difluorophenyl)-5-[2-(morpholine sulfonyl)-2,6,-dihydropyrrolo[3,4-c] pyrazol-5(4H)-yl]tetrahydro-2H-pyran-3-amine

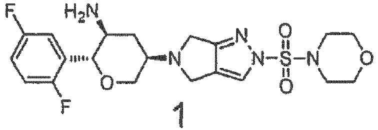

At room temperature, the intermediates [(2R, 3S)-5-oxo-2-(2,5-difluorophenyl)tetrahydro-2H-pyran-3-yl] tert-butyl carbamate (101mg, 0.31mmol) and 2-(N-sulfonyl morpholine)-2,4,5,6,-tetrahydropyrrolo[3,4-c] pyrazol (80mg, 0.31mmol) were dissolved in 6mL anhydrous methanol, 11.4mg decaborane was added at room temperature, stirred overnight, concentrated to dry under vacuum, then 4mL dichloromethane, 1 mL trifluoroacetic acid were added, stirred at room temperature for 2h, concentrated and subjected to column chromatography (methanol:dichloromethane=1:20) to obtain 101mg white solid. ¹H NMR(400MHz, MeOD) δ 7,87(s, 1H), 7.30-7.05(m, 3H), 4.37-4.27(m, 2H), 4.00-3.86(m, 4H), 3.76-3.64(m, 4H), 3.43(t, *J* = 10.7Hz, 1H), 3,31-3.23(m, 4H), 3.13-3.04(m, 1H), 2.99-2.91(m, 1H), 2.54-2.46(m, 1H), 1.54(q, *J*= 11.7Hz, 1 H); ESI-HRMS: calcd for C₂₀H₂₆F₂N₅O₄S, 470.1674, found, 470.1667.

### Example 2

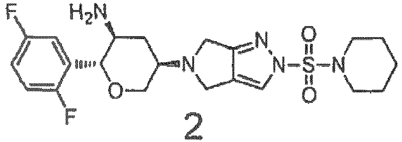

### Preparation of compound 2: (2R, 3S, 5R)-2-(2,5-difluorophenyl)-5-[2-(piperidine sulfonyl)-2,6- dihydropyrrolo[3,4,-c]pyrazol-5(4H)-yl]tetrahydro-2H-pyran-3-amine

The synthetic method of compound 2 of the present invention refers to example 1, the morpholinesulfonyl chloride in example 1 was replaced by piperidine sulfonyl chloride, to obtain a white solid. ¹H NMR(400MHz, DMSO-d₆) δ 7.89(s, 1H), 7.21(m, 3H), 4.16(d, *J*= 9.6Hz, 1 H), 4.12.4.11(m, 1 H), 3.76(d, *J* = 15.2Hz, 4H), 3.26(t, *J* = 10.6Hz, 1 H), 3.17-3.14(m, 4H), 2.95-2.86(m, 2H), 2.34-2.31(m, 1H), 1.50-1.37(m, 7H).ESI-MS: 468.3(M+1).

### Example 3

### Preparation of compound 3: (2R, 3S, 5R)-2-(2,5-difluorophenyl)-5-[2-(pyrrole sulfonyl)-2,6-dihydropyrrolo[3,4,-c]pyrazol-5(4H)-yl]tetrahydro-2H-pyran-3-amine

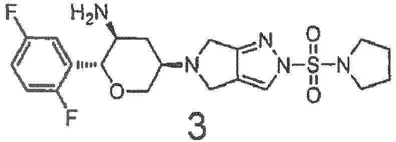

The synthetic method of compound 3 of the present invention refers to example 1, the morpholinesulfonyl chloride in example 1 was replaced by pyrrole sulfonyl chloride, to obtain a faint yellow solid. ¹H NMR(400MHz, DMSO-d₆) δ 8.28(s, 2H), 8.10(s, 1H), 7.37-7.31(m, 3H), 4.59(d, *J*=10.0Hz, 1H), 4.42-4.26(m, 5H), 3.54-3.52(m, 3H), 3.35(t, *J* = 6.4Hz, 4H), 2.67-2.64(m, 1H), 1.89(dd, *J* = 23.4, 11.6Hz, 1H), 1.76-1.73(m, 4H).ESI-MS: 454.7(M+1).

### Example 4

### Preparation of compound 4: (2R, 3S, 5R)-2-(2,5-difluorophenyl)-5-[2-(N-methylpiperazine sulfonyl)-2,6-dihydropyrrolo[3,4,-c]pyrazol-5(4H)-yl]tetrahydro-2H-pyran-3-amine

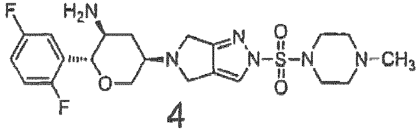

The synthetic method of compound 4 of the present invention refers to example 1, the morpholinesulfonyl chloride in example 1 was replaced by N- methylpiperazine sulfonyl chloride, to obtain a white solid. ¹H NMR(400MHz, MeOD) δ 7,86(s, 1 H), 7.25-7.14(m, 3H), 4.37(d, *J*=9.4Hz, 1 H), 4,31-4.29(m, 1H), 3.92-3.90(m, 4H), 3.44(t, *J*=10.5Hz, 1H), 3.33(br s, 4H), 3.12-3.20(m, 2H), 2.50(br s, 5H), 2.30(s, 3H), 1.58(dd, *J*=23.2, 11.7Hz, 1H). ESI-MS: 483.1 (M+1).

### Example 5

### Preparation of compound 5: (2R, 3S,

5R)-2-(2,5-difluorophenyl)-5-[2-(4-cyclopentylpiperazine-1-yl-sulfonyl)-2,6-dihydropyrrolo[3,4,-c]pyrazol-5(4H)-yl]tetrahydro-2H-pyran-3-amine

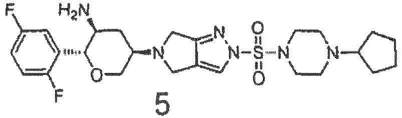

The synthetic method of compound 5 of the present invention refers to example 1, the morpholinesulfonyl chloride in example 1 was replaced by 4-cyclopentylpiperazine-1-yl-sulfonyl chloride, to obtain a white solid. ¹H NMR(400MHz, MeOD) δ 7.86(s, 1H), 7.26-7.10(m, 3H), 4.33(d, *J* = 9.6Hz, 1 H), 4.30-4.28(m, 1 H), 3.91-3.90(m, 4H), 3.43(t, *J*=10.7Hz, 1 H), 3.33(br s, 4H), 3.10-3.04(m, 1H), 3.01-2.95(m, 1 H), 2.58-2.49(m, 6H), 1.92-1.87(m, 2H), 1.74-1.70(m, 2H), 1.62-1.50(m, 3H), 1.41-1.31(m, 2H).ESI-MS: 537.4(M+1).

### Example 6

### Preparation of compound 6: (2R, 3S, 5R)-2-(2,5-difluorophenyl)-5-[2-(3-oxopiperazine -1-yl-sulfonyl)-2,6-dihydropyrrolo[3,4,-c]pyrazol-5(4H)-yl]tetrahydro-2H-pyran-3-amine

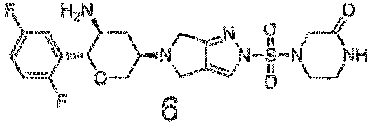

The synthetic method of compound 6 of the present invention refers to example 1, the morpholinesulfonyl chloride in example 1 was replaced by 3-oxopiperazine -1-yl-sulfonyl chloride, to obtain a white solid. ESI-MS:483.1(M+1).

### Example 7

### Preparation of compound 7: (2R, 3S, 5R)-2-(2,5-difluorophenyl)-5-[2-(3-hydroxyazetidine-1-sulfonyl)-2,6-dihydropyrrolo[3,4,-c]pyrazol-5(4H)-yl]tetrahydro-2H-pyran-3-amine

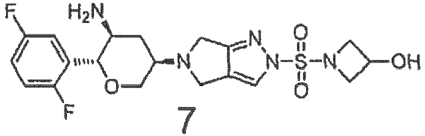

The synthetic method of compound 7 of the present invention refers to example 1, the morpholinesulfonyl chloride in example 1 was replaced by 3-hydroxyazetidine-1-sulfonyl chloride, to obtain a white solid. ESI-MS:456.1(M+1).

### Example 8

### Preparation of compound 8: (2R, 3S, 5R)-2-(2,5-difluorophenyl)-5-[2-(1-isopropylpiperidine-4-sulfonyl)-2,6-dihydropyrrolo[3,4,-c]pyrazol-5(4H)-yl]tetrahydro-2H-pyran-3-amine

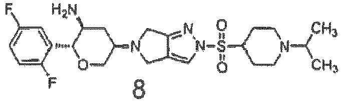

The synthetic method of compound 8 of the present invention refers to example 1, the morpholinesulfonyl chloride in example 1 was replaced by 1-isopropylpiperidine-4-sulfonyl chloride, to obtain a faint yellow solid. ESI-MS:510.2(M+1).

### Example 9

### Preparation of compound 9: (2R, 3S, 5R)-2-(2,5-difluorophenyl)-5-[2-(4-(methylsulfonyl) piperazine-1-sulfonyl)-2,6-dihydropyrrolo[3,4,-c]pyrazol-5(4H)-yl]tetrahydro-2H-pyran-3-amine

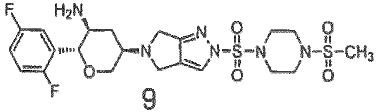

The synthetic method of compound 9 of the present invention refers to example 1, the morpholinesulfonyl chloride in example 1 was replaced by 4-(methylsulfonyl) piperazine-1-sulfonyl chloride, to obtain a white solid. ESI-MS:547.5(M+1).

### Example 10

### Preparation of compound 10: (2R, 3S, 5R)-2-(2,5-difluorophenyl)-N-ethyl-5-[2-(morpholine sulfonyl)-2,6-dihydropyrrolo[3,4,-c]pyrazol-5(4H)-yl]tetrahydro-2H-pyran-3-amine

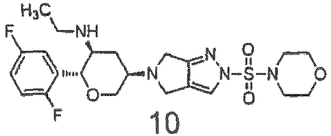

Compound 10 of the present invention was obtained through the reaction of compound 1 with bromoethane under basic condition, it is a light yellow solid. ESI-MS:498.5(M+1).

### Example 11

### Preparation of compound 11: (2R, 3S, 5R)-2-(2,5-difluorophenyl)-N,N-diisopropyl-5-[2-(morpholine sulfonyl)-2,6-dihydropyrrolo[3,4,-c]pyrazol-5(4H)-yl]tetrahydro-2H-pyran-3-amine

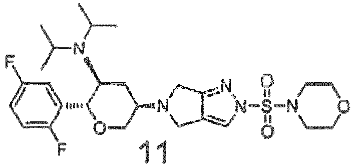

Compound 11 of the present invention was obtained through the reaction of compound 1 with isopropyl bromide under basic condition, it is a grey solid. ESI-MS:554.3(M+1).

### Example 12

### Preparation of compound 12: (2R, 3S, 5R)-2-(2,5-difluorophenyl)-N,N-dibutyl-5-[2-(morpholine sulfonyl)-2,6-dihydropyrrolo[3,4,-c]pyrazol-5(4H)-yl]tetrahydro-2H-pyran-3-amine

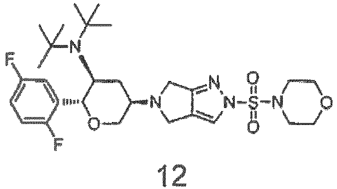

Compound 12 of the present invention was obtained through the reaction of compound 1 with butyl bromide under basic condition, it is a white solid. ESI-MS:581.6(M+1).

### Example 13

Preparation of compound 13: (2R, 3S, 5R)-2-(2,5-difluorophenyl)-5-[2-(azetidine-1-sulfonyl)-2,6-dihydropyrrolo[3,4,-c]pyrazol-5( 4H)-yl]tetrahydro-2H-pyran-3-amine

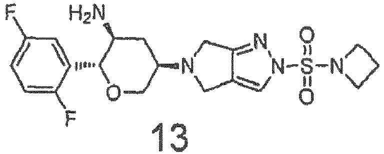

The synthetic method of compound 13 of the present invention refers to example 1, the morpholinesulfonyl chloride in example 1 was replaced by azetidine-1-sulfonyl chloride, to obtain a white solid. ¹H NMR(400MHz, MeOD) δ 7.82(s, 1H), 7.23-7.04(m, 3H), 4.34-4.28(m, 2H), 4.06(t, *J*=7.8Hz, 4H), 3.96-3.92(m, 4H), 3.46(t, *J*=10.7Hz, 1H), 3.15-3.07(m, 1H), 3.01-2.94(m, 1 H), 2.53-2.49(m, 1 H), 2.23-2.15(m, 2H), 1.56(q, *J*= 11.7Hz, 1H). ESI-MS: 440.4(M+1).

### Example 14

### Preparation of compound 14: (2R, 3S, 5R)-2-(2,5-difluorophenyl)-5-[2-(4-methyl piperazine-1-sulfonyl)-2,6-dihydropyrrolo[3,4,-c]pyrazol-5(4H)-yl]tetrahydro-2H-pyran-3-a mine.

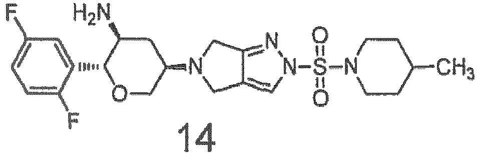

The synthetic method of compound 14 of the present invention refers to example 1, the morpholinesulfonyl chloride in example 1 was replaced by 4-methyl piperazine-1-sulfonyl chloride, to obtain a white solid. ¹H NMR(400MHz, MeOD) δ 7.89(s, 1H), 7.26-7.17(m, 3H), 4.16-4.11 (m, 2H), 3.76(d, *J*=14.5Hz, 4H), 3.70-3.67(m, 2H), 3.26(t, *J*=10.6Hz, 1 H), 2.94-2.83(m, 2H), 2.66(t, *J* = 11.7Hz, 2H), 2.34-2.31 (m, 1H), 1.65-1.62(m, 2H), 1.45-1.36(m, 2H), 1.11-1.01(m, 2H), 0.84(d, *J*=6.5Hz, 3H).ESI-MS:482.1(M+1).

### Example 15

### Preparation of compound 15: (2R, 3S, 5R)-2-(2,5-difluorophenyl)-5-[2-(perhydroindole-1 sulfonyl)-2,6-dihydropyrrolo[3,4,-c]pyrazol-5(4H)-yl]tetrahydro-2H-pyran-3-amine.

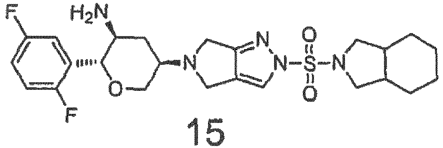

The synthetic method of compound 15 of the present invention refers to example 1, the morpholinesulfonyl chloride in example 1 was replaced by perhydroindole-1-sulfonyl chloride, to obtain a white solid. ¹H NMR(400MHz, MeOD) δ 7,92(s, 1H), 7.27-7.18(m, 3H), 4.20(d, *J*=9.6Hz, 1 H), 4.15-4.12(m, 1 H), 3.76(d, *J*=12.1Hz, 4H), 3.40-3.36(m, 3H), 3.27(t, *J* = 10.8Hz, 2H), 3.22-3.18(m, 3H), 2.99-2.90(m, 2H), 2.36-2.33(m, 1 H), 2.15-2.12(m, 2H), 1.45-1.35(m, 6H). ESI-MS: 508.2(M+1)

### Biological evaluation

### Test 1

Test of the inhibition activity of the compound of the present invention to the DPP-4 enzyme:
Suitable amount of DPP-4 enzyme (SIGMA), HEPES buffer solution from a sample box that was subjected to three times gradient dilution were added in the reaction system, blank control (which does not contain enzyme and sample box), negative control (which does not contain sample) and positive control (positive control is MK-3102) were provided simultaneously, the reaction was carried out at room temperature for 10 minutes, then the substrate Gly-Pro-7-amido-4-methylcoumarin (SIGMA) was added, the reaction was carried out for 30 minutes at room temperature and avoid light, fluorescence was tested, excitation wavelength was 355nm, emission wavelength was 460nm.

Inhibition ratio was calculated according to the measurements of fluorescence, inhibition ratio=[1-(sample-blank)/(negative-blank)]*100%, IC₅₀ value was calculated by 4ParameteriLogistic Model of Xlfit software. The experiment results are shown in table 1.

**Table 1: Test results of activities of the compounds of the examples and the comparative compounds such as Sitagliptin etc to DPP-4 in vitro.**

| Compound No. | DPP-4 inhibition IC₅₀ (nM) | Drugs on the market or compounds from documents | DPP-4 Inhibition IC₅₀ (nM) |
|---|---|---|---|
| 1 | 0.5 | Sitagliptin | 15 |
| 2 | 0.8 | Vildagliptin | 2.8 |
| 3 | 0.3 | 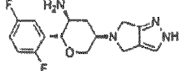 | 1.6 |
| 4 | 0.6 | MK-3102 | 2.2 |
| 5 | 0.2 | 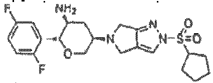 | 1.8 |
| 13 | 0.5 | 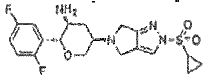 | 1.4 |
| 14 | 0.4 | | |
| 15 | 0.7 | | |

| | | | |
|---|---|---|---|
| Conclusion: the compounds of the present invention have significant inhibition effect to DPP-4 enzyme. | | | |

In another aspect, in the stability test of human liver microsomes, compared with MK-3102, the compounds 1,4 of the present invention and so on have better metabolism stability, indicating that the compounds of the present invention have the prospect of becoming long-term antidiabetic medicine.

## Claims

1. A compound of formula (I): 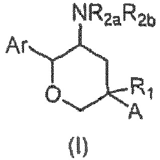 Wherein,
Ar is a substituted or unsubstituted aryl, heteroaryl;
R₁ is a hydrogen, a substituted or unsubstituted alkyl;
R₂ₐ and R_{2b} are each independently selected from hydrogen, or
R₂ₐ and R_{2b} are each independently selected from substituted or unsubstituted alkyl, alkoxy, cycloalkyl, heterocyclylalkyl; or
R₂ₐ and R_{2b} form heterocyclylalkyl with the nitrogen atom attached to them, and the heterocyclylalkyl can be optionally substituted;
A is selected from: 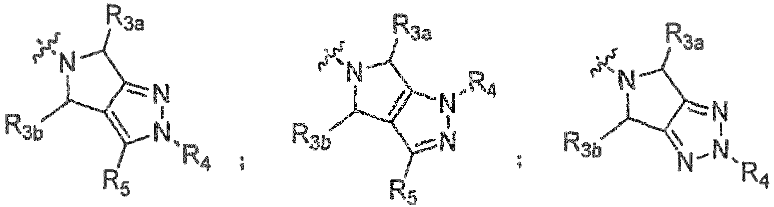 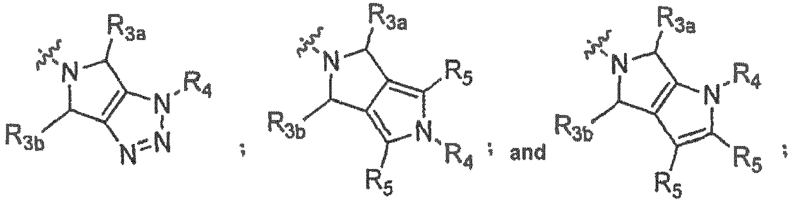
R₃ₐ and R_{3b} are each independently selected from hydrogen, substituted or unsubstituted C₁₋₁₀ alkyl;
R₄ is: 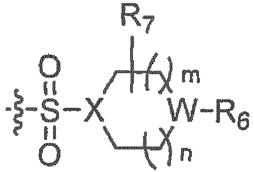
X is CR₈ or N;
wherein 0-3 R₇ may be present, and each R₇ is idependently oxo, halogen, substituted or unsubstituted alkyl, substituted or unsubstituted alkoxy, or two or more R₇ form brideged alkyls;
W is CR₈, O, or N;
R₆ is selected from H, hydroxyl, or
R₆ is selected from substituted or unsubstituted C₁₋₈ alkyl, C₁₋₈ alkyl-R₉, C₃₋₁₄ cycloalkyl, 3-14 membered heterocyclylalkyl, C(O)-C₁₋₈ alkyl, C₁₋₈ halogen substituted alkyl, C₁₋₈ alkyl hydroxyl, C(O)NR₉R₁₀, C₁₋₈ cyano alkyl, C(O)R₉, C₀₋₈alkyl-C(O)-C₀₋₈ alkyl-NR₉R₁₀, C₀₋₈ alkyl-C(O)OR₉, NR₉R₁₀, SO₂-C₁₋₈ alkyl, C₁₋₈ alkyl-C₃₋₁₄ cycloalkyl, C(O)-C₁₋₈ alkyl-C₃₋₁₄ cycloalkyl, C₁₋₈ alkoxy;
R₈ is a hydrogen or halogen;
R₉ and R₁₀ are each independently selected from H, C₁₋₈ alkyl, C₃₋₁₄ cycloalkyl, 3-14 membered heterocyclylalkyl, C₆₋₁₄ aryl, 5-14 membered heteroaryl, alkoxy, C(O)C₁₋₄alkyl, C₁₋₈ alkylamino, C₁₋₆ alkyls hydroxyl;
m is 0, 1 or 2;
n is 0, 1 or 2;
R₅ is selected from H, halogen, cyano, amino, nitro, or
R₅ is selected from substituted or unsubstituted C₁₋₁₀ alkyl, C₁₋₁₀ alkoxy, C₂₋₁₀ alkenyl, (CH₂)ₚ-aryl, (CH₂)ₚ-heteroaryl, (CH₂)ₚ-cycloalkyl, (CH₂)ₚ-heterocyclylalkyl, (CH₂)ₚ-NR₁₁R₁₂, (CH₂)p-SO₂NR₁₁R₁₂, (CH₂)ₚ-SO₂R₁₃, (CH₂)ₚ-NR₁₁SO₂R₁₃, (CH₂)ₚ-OR₁₃, (CH₂)ₚ-OCOR₁₃, (CH₂)ₚ-OCONR₁₁R₁₂, (CH₂)ₚ-CONR₁₁R₁₂, (CH₂)ₚ-NR₁₃CONR₁₁R₁₂, (CH₂)ₚ-COOH, (CH₂)ₚ-COR₁₃, (CH₂)ₚ-CO₂C₁₋₆ alkyl, (CH₂)ₚ-NR₁₁COOR₁₃;
wherein, R₁₁ and R₁₂ are each independently selected from hydrogen, (CH₂)_{q}-phenyl, (CH₂)_{q}-C₃₋₈ cycloalkyl, C₁₋₆ alkyl, wherein alkyl is optionally substituted by 1-5 substituents selected from fluorine or hydroxyl, wherein phenyl and cycloalkyl are optionally substituted by 1-5 substituents independently selected from halogen, hydroxyl, trifluoromethyl, C₁₋₆ alkyl or C₁₋₆ alkoxy;
or R₁₁ and R₁₂, together with the nitrogen atom they are attached to, form a heterocyclic ring selected from piperidine, piperazine, morpholine, pyrrole or azetidine, wherein the heterocyclic ring is optionally substituted by 1-3 substituents independently selected from halogen, hydroxyl, C₁₋₆ alkyl or C₁₋₆ alkoxy;
each R₁₃ is independently C1-6 alkyl, wherein the alkyl is optionally substituted by 1-5 substituents selected from fluorine or hydroxyl;
p is 0, 1, 2, 3, 4, 5 or 6;
q is 0, 1 or 2;
or the pharmaceutical acceptable salts, hydrates, solvates or stereisomers thereof.

2. The compound according to claim 1, wherein, Ar is a phenyl that is optionally substituted by 1-5 R₁₄;
each R₁₄ is independently selected from halogen, hydroxyl, cyano, nitro, nitroso, amino, imino, carboxyl, sulfydryl or
each R₁₄ is independently selected from substituted or unsubstituted alkyl, acyl, acylamino, ester group, acylester group, phenoxy, benzyl, benzyloxy, sulfonyl, sulfinyl, cycloalkyl, heterocyclylalkyls, alkenyl, alkynyl, alkoxy, allyloxy, alkylamino, aryl, heteroaryl.

3. The compound according to claim 2, wherein, Ar is a phenyl that is optionally substituted by 1-3 substituents independently selected from F, Cl, Br, I, -CH₃, -CF₃ and -OCF₃.

4. The compound according to claim 3, wherein, Ar is 2,5-difluorophenyl or 2,4,5-trifluorophenyl.

5. The compound according to claim 1, wherein, R₂ₐ and R_{2b} are each independently selected form hydrogen, C₁₋₁₀ alkyl, alkoxy, C₃₋₁₄ cycloalkyl, 3-14 membered heterocyclylalkyl;
wherein, alkyl is optionally substituted by 1-6 substituents that are independently selected from halogen, hydroxyl, trifluoromethyl;
alkoxyl is optionally substituted by 1-6 substituents that are independently selected from halogen or hydroxyl;
cycloalkyl is optionally substituted by 1-3 substituents that are independently selected from halogen, hydroxyl, cyano, nitro, carboxyl, C₁₋₆ alkyl, C₁₋₆ alkyloxycarbonyl or C₁₋₆ alkoxyl, wherein alkyl and alkoxyl can be substituted by 1-5 fluorine;
heterocyclylalkyl is optionally substituted by 1-3 substituents that are independently selected from oxo, halogen, hydroxyl, cyano, nitro, carboxyl, C₁₋₆ alkyl, C₁₋₆ alkyloxycarbonyl or C₁₋₆ alkoxyl;
or R₂ₐ and R_{2b}, together with the nitrogen atom they are attached to, form a heterocyclic ring that is selected from piperidine, piperazine, morpholine, pyrrole or azetidine, wherein the heterocyclic ring is optionally substituted by 1-3 substituents that are independently selected from halogen, hydroxyl, C₁₋₆ alkyl or C₁₋₆ alkoxyl, wherein each alkyl and alkoxyl are optionally substituted by 1-5 fluorine.

6. The compound according to claim 5, wherein, R₂ₐ and R_{2b} are each independently selected from hydrogen, C₁₋₆ alkyl that is optionally substituted by 1-3 fluorine or hydroxyl; or
R₂ₐ and R_{2b}, together with the nitrogen atom they are attached to, form a heterocyclic ring that is selected from piperidine, piperazine, morpholine, pyrrole or azetidine.

7. The compound according to claim 6, wherein, R₂ₐ and R_{2b} are hydrogens.

8. The compound according to claim 1, wherein, R₃ₐ and R_{3b} are each independently selected from hydrogen, C₁₋₆ alkyl that is optionally substituted by 1-6 fluorine.

9. The compound according to claim 8, wherein, R₃ₐ and R_{3b} are hydrogens.

10. The compound according to claim 1, wherein, A is:

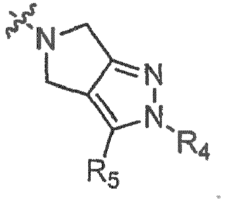

11. The compound according to claim 10, wherein, R₅ is hydrogen.

12. The compound according to claim 1, wherein, R₄ is: 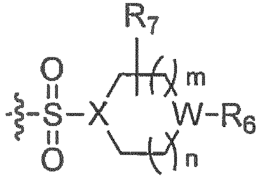
X is CR₈ or N;
wherein 0-2 R₇ may be present, and R₇ is oxo, or two R₇ form brideged alkyl;
W is CR₈, O, or N;
R₆ is selected from H, hydroxyl, or
R₆ is selected from substituted or unsubstituted C₁₋₈ alkyl, C₃₋₈ cycloalkyl, 3-8 membered heterocyclylalkyl, C(O)-C₁₋₆ alkyl, C(O)NR₉R₁₀, C(O)R₉, NR₉R₁₀, SO₂-C₁₋₈alkyl;
R₈ is a hydrogen or halogen;
R₉ and R₁₀ are each independently selected from H, C₁₋₆ alkyl, C₃₋₈ cycloalkyl, 3-8 membered heterocyclylalkyl;
m is 0, 1, or 2;
n is 0, 1 or 2.

13. The compound according to any one of claims 1-12, wherein, the compound of formula I is shown as structure formula la or Ib, which has the shown absolute stereochemical configuration on the two * marked carbon atoms that are formed stereoscopically:

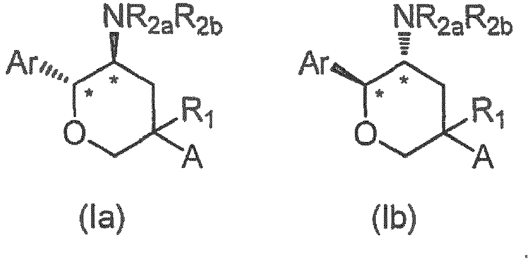

14. The compound according to claim 13, wherein, the compound of formula I is shown as structure formula la, which has the shown absolute stereochemical configuration on the two * marked carbon atoms that are formed stereoscopically:

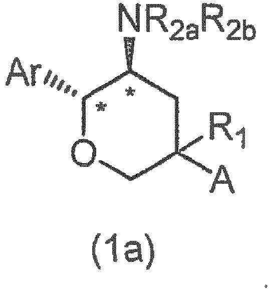

15. The compound according to claim 14, wherein, the compound of formula Ia is shown as structure formula Ic and Id, which have the shown absolute stereochemical configuration on the two * marked carbon atoms that are formed stereoscopically:

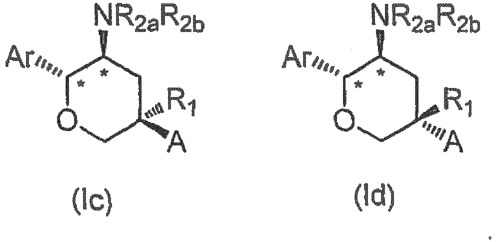

16. The compound according to claim 15, wherein, the compound of formula la is shown as structure formula Ic, which has the shown absolute stereochemical configuration on the two * marked carbon atoms that are formed stereoscopically:

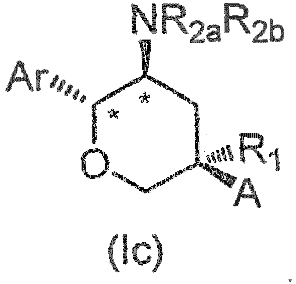

17. The compound according to claim 16, wherein A is selected from:

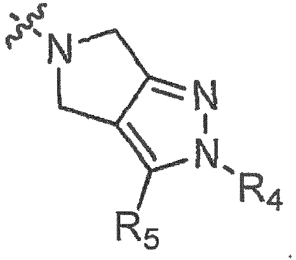

18. The compound according to claim 17, wherein R₅ is H.

19. The compound according to claim 1, which is selected from the following compounds:

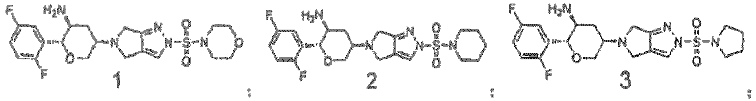

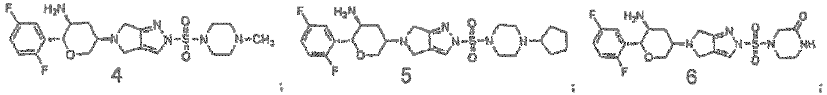

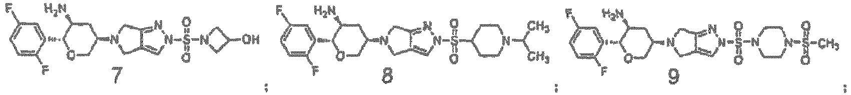

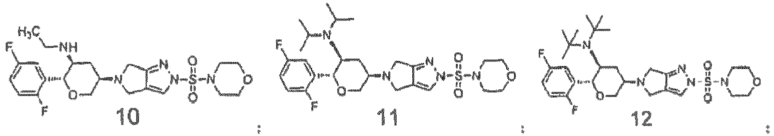

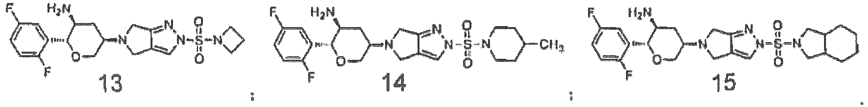

20. A method for treating disease, disorder or syndrome related to inhibition of DPP-4 comprising administering the compound according to any one of claims 1-19 or the pro-drugs thereof or the pharmaceutical compositions comprising the compound of formula (I) or the pro-drugs thereof and the pharmaceutically acceptable excipients to an individual in need thereof.

21. The method according to claim 20, wherein the disease, disorder or syndrome is selected from insulin resistance, hyperglycemia, type 2 diabetes, wherein the individual comprises human.
